# EUROPEAN PATENT APPLICATION

(11) **EP 1 127 495 A1**
(43) Date of publication of application: **29.08.2001**
(21) Application number: 99954391.1
(22) Date of filing: 05.11.1999
(51) Int. Cl.: A23L 1/20, A23L 1/10, B02C 19/06, B02C 25/00

(54) **METHOD FOR CLASSIFYING SPECIFIC TISSUE OF OILSEEDS OR CEREALS AND FINELY MILLED POWDERS**

(30) Priority: 05.11.1998 JP 31415098; 27.11.1998 JP 33668898
(71) Applicant: Ajinomoto Co., Inc., Tokyo 104-8315 (JP)
(72) Inventor: OZAWA, Yoichi, Food R&D Lab., Kawasaki-shi, Kanagawa 210-8681 (JP); UENO, Goro, Ajinomoto Oil Mills Co., Inc., Yokohama-shi, Kanagawa 2300-0053 (JP); NAKATANI, Akihiro, Ajinomoto Oil Mills Co., Inc., Yokohama-shi, Kanagawa 2330-0053 (JP); YOSHIDA, Teruo, Food R&D Lab., Kawasaki-shi, Kanagawa 210-8681 (JP); MORINAGA, Yasushi, Food R&D Lab., Kawasaki-shi, Kananagawa 210-8681 (JP)
(74) Representative: Strehl Schübel-Hopf & Partner
(86) International application number: JP9906159
(87) International publication number: WO0027222

(57) **Abstract**

The purpose of the present invention is to classify tissues of oilseeds and cereals into the fractions of fine structure portions so that physicochemical properties that are not sufficiently apparent in the whole tissues may clearly appear.

The purpose of the present invention is to provide ultra-finely crushed powder that is much more advantageous in the following aspects:
1) properties as food stuff such as smoothness, water holding capacity, suspension-state holding capacity;
2)sterilization efficiency of the suspension and transportation efficiency in the pipe line when used as material for fermentation;
3) extraction efficiency of various components contained in the ultra-finely crushed powder;
4) reaction efficiency with enzymes, digestion and absorption efficiency, and feed efficiency when used as feed component;
5) quality of containers such as a food tray having a good biodegradablity, which comprises the ultra-finely crushed powder of the present invention as filler.

The present invention relates to a method for producing a fraction derived from the fine structure portions of a specific tissue of oilseeds or cereals, comprising crushing and classifying material of the specific tissue.

The present invention relates to an ultra-finely crushed powder having 50 % grain size of 6 µm or less, which is obtained from the material of a specific tissue of oilseeds or cereals.

The present invention relates to a method for producing ultra-finely crushed powder having 50 % grain size of 10 µm or less, comprising crushing material of the specific tissue of oilseeds or cereals in a dry state.

## Description

### Technical Field

The present invention relates to a method for producing a fraction derived from the fine structure portions of a specific tissue such as hulls, germ, and cakes obtained by fat extraction or protein extraction of oilseeds (seeds rich in oil and fat) or cereals, comprising crushing material of the specific tissue and classifying the same.

The invention further relates to ultra-finely crushed powder having 50% grain size (the grain size that corresponds to 50% of an accumulated distribution curve of each grain size, which is the same as "50% diameter D₅₀") of 6 µm or less, to a method for producing ultra-finely crushed powder having 50% grain size of 10 µm or less by crushing them in a dry state, and various composition comprising the ultra-finely crushed powder.

### Background Art

A huge amount of oilseeds such as soybean, rapeseed, corn, and wheat is imported from abroad every year for the production of oil and fat, protein, starch and the like. Their cotyledon and albumen are mainly used for the above production. Oil component is usually extracted with n-hexane from cotyledon, albumen and germ. Soybean protein is further extracted from the resulting extraction cake with water.

The technique called a "dry milling" is well known for separating in a dry state corn or wheat starch from their albumen, or soybean protein from cotyledon of the whole soybean or defatted soybean.

The Japanese Patent No. 2,803,853 discloses a method for the separation of a protein-rich fraction and a fiber-rich fraction from wheat bran, in which the wheat bran was pulverized and classified into a fraction with a grain size of 300 ± 25 µm or less and 100 ± 25 µm or more, and a fraction with a grain size of 100 ± 25 µm or less.

The Japanese Patent Application laid open No.Hei.7-265000 discloses ultra-finely crushed powder that was obtained by preliminarily crushing a bran fraction of wheat, barley, rye, oat, adlay, sorghum and the like by means of a cutting and shearing mill, followed by the crushing with a jet mill-type crusher or a shearing mill. However, it is apparent that 50% grain size of the ultra-finely crushed powder was ten and a few microns or more as judged from a grain distribution chart described in the above reference.

on the other hand, materials derived from other tissues than the above bran, or the extraction cake of cotyledon, albumen and germ are commercially utilized as feed and fertilizers at a low price. Those materials are also used as a starting material for the extraction of polysaccharides (for example, the Japanese Patent Application laid open No.Sho.64-62303 and the Japanese Patent Application laid open No.Hei.5-262802). And, they are finely milled to be used as food stuff (for example, the Japanese Patent Publication No.Hei.3-69270 in which corn fiber was crushed into particles of 100 microns or less with a colloid mill-type mass colloider in a wet state; and the Japanese Patent Application laid open No.Hei.3-67595 in which raw "okara" or soybean pulp (a solid by-product in the production of soybean curd "tofu") was crushed into particle with an average diameter of 25 microns or less with a homogenizer in a wet state).

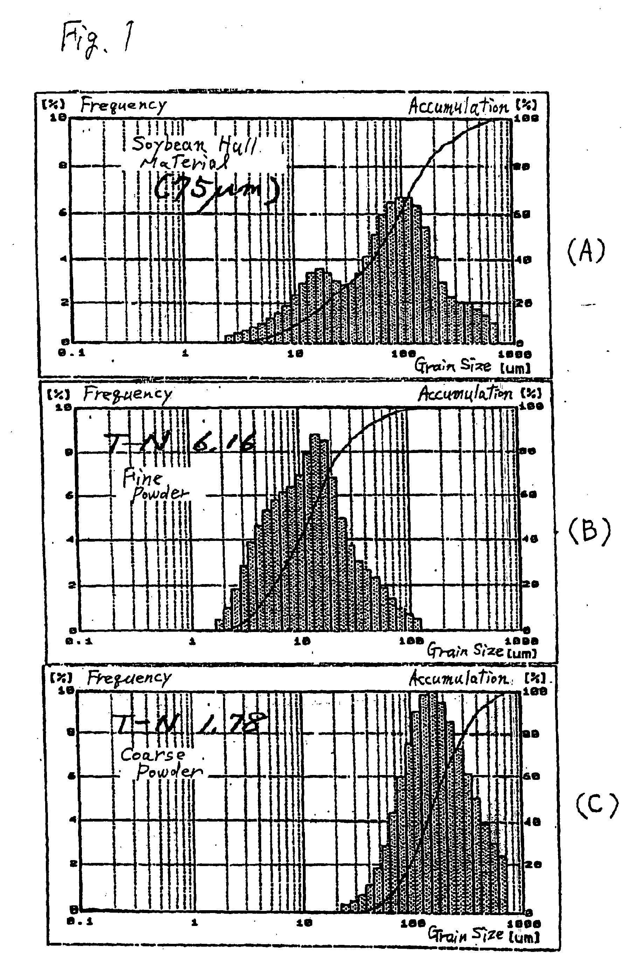

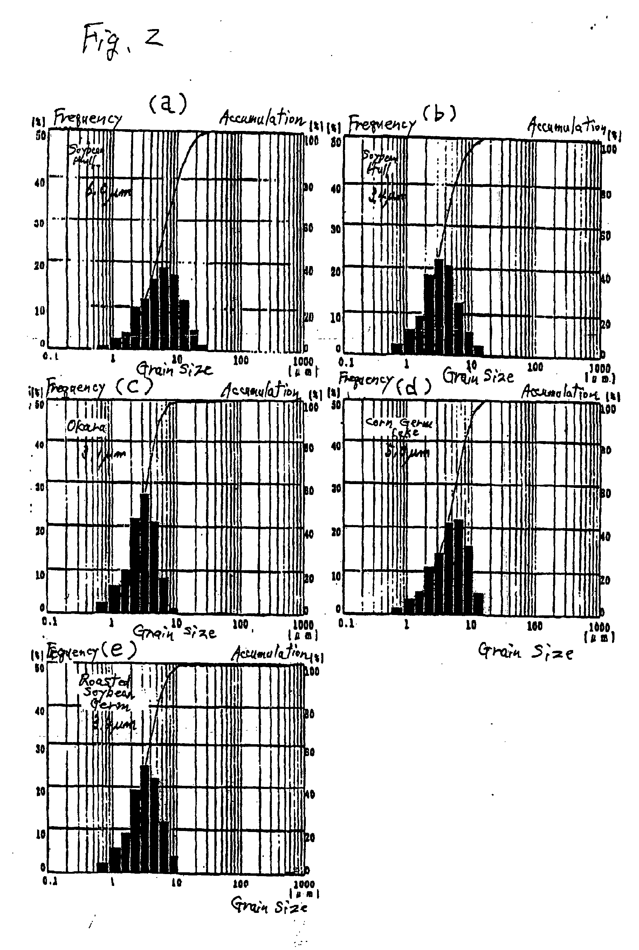

The Japanese Patent Publication No.Hei.3-69270 discloses an example wherein the corn fiber was crushed into 100 microns in length at its maximum measuring direction with the colloid mill-type mass colloider. As seen from the figures 1 - 5 in the same publication, it looks like that 50 % grain size of the crushed material was several tens microns or more.

Further, the Japanese Patent Publication No.Hei.3-67595 discloses an example wherein raw "okara" was crushed by means of the homogenizer into particles with an average grain size of 7.0 microns according to the measurement with a Coulter counter. However, the resulting crushed material is no more than an intermediate aqueous suspension that is obtained as a result of a pretreatment for the acceleration of decomposition of protein and fiber contained in water-insoluble dietary fiber in the processes of the production of water-soluble polysaccharides from water-insoluble dietary fiber. The crushed material was therefore subjected further to the decomposition treatment of protein and fiber contained therein. This means that the crushed material per se will not be used as food stuff and the like.

Thus, although some techniques are already known for finely crushing the specific tissues of oilseeds to be used as food stuff, all of them are carried out in the wet state.

On the other hand, the crushing using a Pinmill-type crusher in a dry state could only produce particles with an average gain size of several tens microns or more. Even the crushing using a ball mill-type crusher that was thought to be most effective in producing fine particles in the dry state could not produce particles with 50 % grain size of ten microns or less when material which is bulky and light or slippery on its surface such as the bean hulls was charged in the dry state. Thus, there is no example in which finely crushed particles with 50 % grain size of ten microns or less are actually obtained in the dry method, either.

According to an observation in a micro scale, the tissues of oilseeds and cereals do not have a uniform structure, but a non-uniform structure such as that having layers (Watanabe, Tokio, Hashizume et al., "Soybean and Its Preparation" Kenko Co., p.6, 20 June 1987). Each of fine structure portions of the non-uniform structure is expected to have different chemical components and to show different physical properties.

Accordingly, if the tissues could be classified into fractions of the above portions, physicochemical properties that are not sufficiently apparent in the whole tissues might clearly appear.

However, except the above examples, there is no prior art that discloses the classification of the specific tissues of oilseeds and cereals into the fractions of fine structure portions.

The present inventors have completed this invention based on the finding that the specific tissue of oilseeds and cereals can be classified into fractions of the fine structure portions in accordance with the differences in their physicochemical properties such as specific volume and grain size pending on sensitivity to crushing, after being crushed into particles with several to several hundreds microns and classified by means of a sorting with air or a sieve.

By the way, it is said that our tongue can not feel roughness any more for particles with the grain size of about 20 microns or less. Accordingly, since the materials with an average grain size of several tens microns feel rough on our tongue and they are inferior in taste and suspending property, opportunities of their use as food stuff have been limited.

When suspension comprising the fine particles with the average grain size of several tens microns is used as a starting material for fermentation, brewing or enzyme reaction, there occur some problems to be resolved for an industrial application. That is, spore-forming bacteria can not be completely sterilized during a sterilization step and sterilization efficiency will become low, and precipitation will occur in a pipe line for transporting the suspension. Further, some precaution are always needed against corruption since the crushing in the prior arts was carried out in the wet state.

Besides, when the material with the average grain size several of tens microns is used as a filler material for shaping a container, the surface of the resulting container will become rough, and its quality will be inferior to a container comprising ultra-fine powder of calcium carbonate as the filler.

The present inventors have tested various methods in order to crush the material of the specific tissue of oilseeds and cereals into particles with 50 % gain size of 6 microns or less. As a result, we have succeeded in crushing the above materials into ultra-finely crushed powder having 50% grain size of 6 µm or less, by using a method in which the materials collide to the wall of an apparatus or with each other by means of a high-speed compressed air or gas (Ultra-fine Jet Pulverizer (Kurimoto Ltd.), a jet miser a micronizer, and Kriptron (Kawasaki Juko Co.), and the like).

### Disclosure of the Invention

The present invention relates to a method for producing a fraction derived from the fine structure portions of a specific tissue of oilseeds or cereals, comprising crushing and classifying material of the specific tissue.

The term "specific tissue of oilseeds or cereals" means in this specification any tissues other than cotyledon, albumen, defatted soybean and wheat bran, which may be classified into their fine structure portions, including, for example, hulls (seed coat), germ, oil and fat-extraction cake, protein-extraction cake and the like.

Their preferred examples include the hulls of soybean, corn outer hull, rape seed, and sesame seed; husk; germs of soybean, corn, wheat, and rice; defatted rice bran; corn germ cake; dry "okara" or soybean pulp; cakes of rape seed and sesame seed.

The material of the specific tissue may be preliminarily dried before the crushing in order to control its water content, or may be used in an intact condition. The materials may be subjected to an appropriate treatment or processing.

Two or more kinds of the specific tissues may be combined at appropriate ratio and used as the material in the present invention.

Crushing may be carried out in this invention by any way and apparatus well known in the art both in a dry and wet state. In the wet state, the material has to be dried after crushing. Usually, the Pinmill-type crusher making use of shearing stress, mass colloider, impact stress-type crusher, and jet a jet miser, are preferably used to crush the material so that the fine structure portions of each specific tissue may be well peeled and separated with each other to give their mixture.

Some kinds of the crushing methods may be combined and used in the present invention. And, crushing conditions such as a degree of the crushing may be selected optionally by those skilled in the art, depending on the kinds of materials, use and application of the desired fraction, the kind of classifying method and the like. For example, the material may be crushed into powder having 50 % grain size of about a few µm - several hundreds µm, i.e., of 5 µm - 500 µm so that their fine structure portions may be separated well.

Classifying may also be carried out in this invention by any way and apparatus well known in the art as long as it utilizes the differences in their physicochemical properties such as specific volume and grain size. For example, it may include the sorting with air and the sieve. The jet mill-type crusher may perform both crushing and classifying simultaneously.

Classifying conditions such as a degree of the classifying (the number of the resulting fractions) may be selected optionally by those skilled in the art, depending on the kinds of materials, use and application of the desired fraction, the kind of crushing method and the like.

Still, as shown by the following examples, it is preferred that the crushed material is classified into a fine fraction having 50 % grain size of a few µm ~ several tens µm, i.e., of 2 µm ~ 50 µm and a high specific volume of 2.5 ml/g ~ 3.1 ml/g, and a coarse fraction having 50 % grain size of several tens µm ~ several hundreds µm, i.e., of 50 µm ~ 500 µm and a low specific volume of 1.6 ml/g ~ 2.3 ml/g, since their physicochemical properties that are not sufficiently apparent in the original whole tissues will very likely appear clearly.

Mixture of one or more kinds of the fractions thus obtained may be used as the material in the present invention to be further crushed and classified.

The present invention is also related to the fraction derived from the fine structure portions of the specific tissue, which is obtained by the above method.

Especially, the fraction having 50 % grain size of a few µm-ten and several µm, i.e., of 2 µm ~ 15 µm is preferred since it has excellent properties.

The fraction according to the present invention is not necessarily derived from one kind of the fine structure portions, but may contain more tow or more kinds of the fine structure portions depending on the crushing and classifying conditions.

The present invention is further related to ultra-finely crushed powder having 50 % grain size of 6 µm or less, preferably 4 µm or less, which is obtained from the material of the specific tissue of the oilseeds or cereals.

The method for crushing of the materials may be optionally selected without any limitation by those skilled in the art as long as the desired 50 % grain size is obtained. As described before, the methods are preferred in which the material collides to the wall of an apparatus or with each other by means of a high-speed compressed air or gas, or by means of rotation at a high speed, using the jet mill-type crusher (Kurimoto Ltd.), the jet miser, the micronizer, and Kriptron (Kawasaki Juko Co.), and the like.

A classifying rotor may be combined with the above apparatus in appropriate conditions to obtain the ultra-finely crushed powder having a desired grain size more effectively.

The invention is therefore related to a method for producing ultra-finely crushed powder having 50 % grain size of 10 µm or less, preferably 6 µm or less, more preferably 4 µm or less, comprising crushing the material of the specific tissue of oilseeds or cereals in a dry state.

Preferably, the crushing is carried out in the dry state by using the high-speed compressed air or gas.

The crushing conditions may be selected optionally by those skilled in the art, depending on the kinds of materials, the desired average grain size, and use and application of the resulting ultra-finely crushed powder.

The materials may be directly crushed, or may be preliminarily crushed with a mechanical crusher such as the Pinmill-type crusher to a grain size of several tens µm ~ several hundreds µm, for example, 50 µm ~ 300 µm so as to shorten the time for crushing into the ultra-finely crushed powder of the present invention.

Mixture of two or more kinds of the materials of the specific tissues may be subjected to the crushing method of the present invention. Alternatively, mixture of two or more kinds of the ultra-finely crushed powder that are derived from different materials may be included in the present invention. Mixing ratios in these mixtures may be optionally selected by those skilled in the art depending on their use and the like.

The ultra-finely crushed powder of the present invention is much more advantageous than the conventional finely-crushed powder having 50 % grain size of several tens µm ~ ten and several µm in the following aspects:
1) properties as food stuff such as smoothness, water holding capacity, suspension-state holding capacity;
2)sterilization efficiency of the suspension and transportation efficiency in the pipe line when used as material for fermentation;
3) extraction efficiency of various components contained in the ultra-finely crushed powder;
4) reaction efficiency with enzymes, digestion and absorption efficiency, and feed efficiency when used as feed component;
5) quality of containers such as a food tray having a good biodegradablity, which comprises the ultra-finely crushed powder of the present invention as filler.

Accordingly, the present invention is also related to various kinds of compositions that comprise the ultra-finely crushed powder of the present invention, such as food stuff for various kinds of food (confectionery, bread, desserts such as ice cream, source, retort pouched food, oil and fat food, emulsified food, liquid food, dietary fiber-enriched food, low-calorie food, low-fat food, nutritional component-enriched food, animal food product, marine product and the like), a culture medium for bacteria in fermentation and brewing, filler for shaping containers including the food trays, and the containers comprising the filler.

### Brief Description of Drawings

Fig.1 shows charts of the grain size distribution of the ultra-finely crushed soybean hull obtained by the treatment with the Pinmill-type crusher two times (50 % grain size:75 µm) (A), the soybean hull-derived "fine" powder obtained by the sorting with air of the ultra-finely crushed soybean hull (A) (50 % grain size:12 µm) (B), and the soybean hull-derived "coarse" powder obtained by the same way (50 % grain size:163 µm)(C). In each chart, a bar graph represents frequency (%) scaled on the left vertical axis, and a line graph represents accumulation (%) scaled on the right vertical axis. A horizontal axis means grain size (µm).
Fig.2 shows charts of the grain size distribution of various kinds of the ultra-finely crushed powder prepared in Example 7.

(a):soybean hull (50 % grain size:6.0 µm); (b):soybean hull (50 % grain size:3.4 µm); (c) dried "Okara" (50 % grain size:3.1 µm); (d):corn germ cake (50 % grain size:5.0 µm); and (e):roasted soybean germ (50 % grain size:3.4 µm). In each chart, a bar graph represents frequency (%) scaled on the left vertical axis, and a line graph represents accumulation (%) scaled on the right vertical axis. A horizontal axis means grain size (µm).

### Best Mode for Carrying Out the Invention

The value of "50% grain size" in the following examples was measured in ethanol by means of MICROTRAC II DRY SYSTEM (NIKKISO). The term "50% grain size" means the grain size that corresponds to 50% of an accumulated distribution curve of each grain size, which is the same as "50% diameter D₅₀".

The T-N (Total Nitrogen) value was measured in Kjeldahl method with KJELTEC AUTO 1030 Analyzer.

The specific volume was determined as follows: the ultra-finely crushed powder was gradually added to a measuring cylinder (500 ml) while being vibrated until a level of 500 ml would not change any more, and the weight of the ultra-finely crushed powder comprised at the level of 500 ml was measured. The specific volume was calculated from the weight thus measured.

The present invention will be specifically described by referring to the following examples, which should not be construed to limit the technical scope of the present invention.

### Example 1

Soybean hull was crushed with a Pinmill type crusher. In the case of Pallmann-Universal Mill PX, the crushing was done at a rotation rate of 17,000/ min. and a charged amount being about 60 kg/ hr. 50% grain size of the resulting crushed powder was 150 µm. The second, third and forth (final) crushing in the same conditions gave the ultra-finely crushed powder derived from the soybean hull having 50% grain size of 75 µm, 43 µm, and 28 µm, respectively (Table 1). They were then classified with the air sorting. The classifying conditions are summarized in Table 2 below. The results of the classification are shown in Table 3.

**[Table 1]**

| | Times of Crushing | 50% Grain Size |
|---|---|---|
| Soybean hull | one | 150 µm |
| | two | 75 |
| | three | (43) |
| | four | 28 |

**[Table 2]**

| Material | 50% Grain Size | Air Rate | Rotation in Sorting | Production Rate |
|---|---|---|---|---|
| Pinmill Crushing (once) | 150 µm | 1 m³/min | 3,000 rpm | 1440 g/h |
| Pinmill Crushing (twice) | 75 | the same as above | 5,000 | 1280 |
| Pinmill Crushing (four times) | 28 | the same as above | 5,000 | 975 |

**[Table 3]**

| | | Fractions by Classification with the Air Sorting | | | | | | | |
|---|---|---|---|---|---|---|---|---|---|
| Material (Soybean hull) | | Fine (light) Powder | | | | Coarse (heavy) Powder | | | |
| | 50% Grain Size | Color | 50 % Grain Size | Yield % | T-N% | Color | 50 % Grain Size | Yield % | T-N% |
| Pinmill | 150 | White- | 26 µm | 37 | 5.65 | Sepia | 223 µm | 63 | 1.64 |
| Crushing (once) | µm | gray | | | | | | | |
| Pinmill | 75 | White- | 12 | 36 | 6.16 | Sepia | 163 | 64 | 1.78 |
| Crushing (twice) | | gray | | | | | | | |
| Pinmill | 28 | White- | 11 | 44 | 4.11 | Sepia | 77 | 56 | 1.31 |
| Crushing (four tiems) | | gray | | | | | | | |

As shown in Table 3, each of the resulting powder was classified into a fine (light) powder fraction and coarse (heavy) powder fraction, each fraction having different values of 50% grain size and T-N. The fact that these two fractions have a different microscopic shape with each other seems to demonstrate the difference in their fine structure. The color of the crushed powder before the air sorting was pale sepia.

Analytical values of the contents of the fine and coarse fractions classified from the ultra-finely crushed powder obtained through the above four crushing procedures with the Pinmill-type crusher are summarized in Table 4. The specific volume of the fine powder was 2.73 ml/g, and that of the coarse powder 2.02 ml/g.

**[Table 4]**

| Sample | Water % | Protein % | Fat & Oil % | Fiber % | Ash % | Sugars % | Dietary Fiber % | Energy |
|---|---|---|---|---|---|---|---|---|
| Fine Powder | 7.2 | 24.0 | 6.2 | 13.0 | 6.8 | 42.8 | 47.4 | 375 kcal |
| Coarse Powder | 6.8 | 8.0 | 1.7 | 37.6 | 4.3 | 41.6 | 76.5 | 364 |
| (Analyzed by Japan Food Analysis Center) | | | | | | | | |

As shown in Table 4, the analytical values of the contents of water, protein, fat and oil, fiber, ash, sugars, and dietary fiber, and of energy were significantly different with each other, which means that the fine structure of the soybean hull was classified according to the present method.

Fig.1 shows the grain size distribution of the soybean hull-derived fine powder (specific volume: 2.84 ml/g) and the soybean hull-derived coarse powder (specific volume: 1.92 ml/g), which were classified with the air sorting from the ultra-finely crushed soybean hull obtained by the treatment with the

Pinmill-type crusher twice. Fig.1 demonstrates that the soybean hull can be classified, and a fraction obtained by the classification can be more finely classified again.

### Example 2

Dry "okara", corn germ cake and soybean germ were used as a starting material of the present invention.

Dry "okara" (freeze-dried raw "okara"), corn germ cake and soybean germ were pre-heated at 80 ° C or more and crushed with the Pinmill-type crusher. The resulting products were then crushed with a jet mill-type crusher (air *pressure*:7kg/cm2, nozzle:3φ, 11,500 rpm) while being classified with air sorting into a fine powder fraction that was discharged from the upper part of the crusher and a coarse powder fraction that remained in it. The results are shown in Table 5.

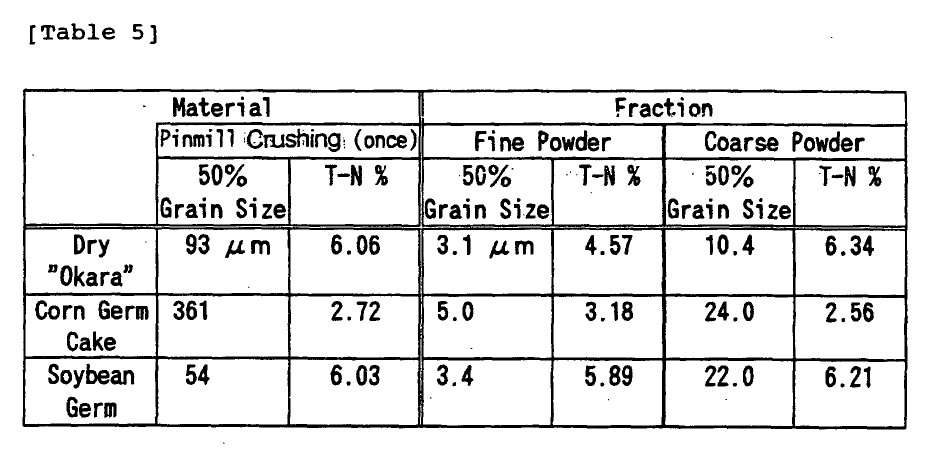

The N-T value of the resulting fine powder was different from that of the starting material, demonstrating that the crushed material was classified into the fine powder and coarse powder that reflected a micro partial structure of the material,

### Example 3

The ultra-finely crushed soybean hull powder (50 % grain size: 75 (µm) obtained by the treatment with the Pinmill-type crusher twice under the same conditions as in Example 1 was classified with various kinds of a vibrating sieve having 60 - 400 meshes for 5 min. The weight of each resulting fraction was determined and their 50 % grain size was calculated. The results are shown in Table 6.

Table 6 shows that peaks in the weight are found in the fractions obtained with 250-400 mesh (50 % grain size: 39 µm) and 80-120 mesh (50 % grain size: 172 µm). If fine powder and coarse powder are divided into two fractions on the borderline of 400 mesh, the ratio between the two fractions will be 31:69, showing that it is possible to carry out the classification with the sieve according to the present invention.

**[Table 6]**

| Meshes | Below 400 | 250-400 | 200-250 | 120-200 | 80-120 | 60-80 | Over 60 | |
|---|---|---|---|---|---|---|---|---|
| Weight % | 3.6 | 27.2 | 12.5 | 18.8 | 19.1 | 12.2 | 6.6 | 100 % |
| 50% Grain Size | 18 | 39 | 64 | 125 | 172 | 247 | 386 | |

### Example 4

The ultra-finely crushed soybean hull powder (50 % grain size: 75 µm) obtained by the treatment with the Pinmill-type crusher twice under the same conditions as in Example 1 was classified with the air sorting according the same conditions as in Example 1 to give a fraction of fine powder (50 % grain size: 12 µm) and a fraction coarse powder (50 % grain size: 163 µm).

Water-soluble protein and polysaccharides were then extracted from the above fractions.

The powder (50 g) was mixed with water (500 ml), adjusted to pH 9, and stirred at a room temperature for 60 min for extraction, followed by centrifugation to remove contaminants. Supernatant was lyophilized to give a solid material. Table 7 shows the extract conditions, extraction rate and T-N values of the extracts.

As shown in Table 7, the fine powder has a higher extraction efficiency (yield) and T-N value than those of the whole part of the crushed soybean hull, demonstrating that the fine powder is more advantageous as a starting material for the extraction of the protein.

**[Table 7]**

| Material | Yield of Extract in Water at pH 9, room temp., 60 min | T - N % |
|---|---|---|
| Crushed Soybean Hull Powder | 12 % | 4.1 |
| Fine powder | 25 | 6.2 |
| Coarse Powder | 8 | 2.3 |

### Example 5

The ultra-finely crushed soybean hull powder (50 % grain size: 75 µm), the fraction of fine powder (50 % grain size: 12 µm) and the fraction of coarse powder (50 % grain size: 163 µm), which were obtained in Example 4, were used as starting materials for the extraction of oil contained therein with n-hexane. The extracted oil was subjected to a thin layer chromatography and gas chromatography to determine a total amount of phytosterol according to a standard oil analysis test (Japan Oil Chemists Society).

The results summarized in Table 8 show that the oil content in the coarse powder was 1.7 %, whereas the total amount of phytosterol was 22.7 % that is about 56 times as high as that of a usual soybean oil. On the other hand, the oil content in the fine powder was 6.2 % that is about 3.6 times as high as that in the coarse powder, but the total amount of phytosterol was about one fifth of that in the coarse powder.

Thus, it is possible to classify the soybean hull crushed powder and to extract a specific and advantageous component locally existing in the hull part such as phytosterol from the classified products in a more effective way at a high concentration. The phytosterol is known to have a function to inhibit the absorption of cholesterol or to promote its discharge from a body. The coarse powder, which correspopnds to the fraction according to the present invention, is advantageous as a starting material for a oil component that is rich in phytosterol. The measurement of the total amount of phytosterol was carried out by JAPAN OILSTUFF INSPECTORS.

**[Table 8]**

| Material | Oil Content % | Total Amount of Phytosterol % |
|---|---|---|
| Crushed Soybean Hull Powder | 3.1 | 7.0 |
| Fine powder | 6.2 | 5.7 |
| Coarse Powder | 1.7 | 8.3 |
| Normal Soybean | 18 - 20 | 0.4 |

### Example 6

Each 20 g of the fraction of fine powder (50 % grain size: 12 µm) and the fraction of coarse powder (50 % grain size: 163 µm), which were obtained in Example 4, was mixed with water (200 ml) and subjected to the extraction with stirring at pH 4.5 and a room temperature for 60 min, followed by centrifugation to remove solid components. The resulting extract was lyophilized and subjected to an amino acid analysis. The results in Table 9 show that the fraction of the coarse powder of the soybean hull according to the present invention is advantageous as a starting material for the extraction of protein that is rich in HO-Pro contained in the soybean hull since it has a significantly high content of HO-Pro in water extract.

**[Table 9]**

| | H0-Pro (mole %) |
|---|---|
| Fine Powder | 0.0 |
| Coarse Powder | 4.1 |
| Soybean Globulin | 0.0 |

### Example 7

The soybean hull was roughly cut into cubes of about 1~3 mm³ and preliminarily crushed with the Pinmill-type crusher. Dry "okara" (lyophilized flakes of raw "okara") and corn germ cake were preliminarily crushed with the Pinmill-type crusher. Roasted soybean germ was prepared by preliminarily heating the soybean germ cake at 80 ° C for 40 min, and preliminarily crushed with the Pinmill-type crusher. These materials were then crushed with Ultra-fine Jet Pulverizer(Kurimoto Ltd.). The crushing conditions and 50 % gain size of the resulting ultra-finely crushed powder are summarized in Table 10.

**[Table 10]**

| Material | | Crushing Conditions | Results |
|---|---|---|---|
| | 50 % Grain Size(*µ*m) | Rotation Rate and Air Pressure | 50 % Grain Size(*µ*m) |
| Soybean Hull | 1 - 3 x 10³ | 10,000 rpm, 7 kg/cm² | 6.0 |
| Soybean Hull | 150 | the same as the above | 3.4 |
| Dry "Okara" | 93 | 11,500 rpm, 7 kg/cm² | 3.1 |
| Corn Germ Cake | 361 | 6,000 rpm, 8 kg/cm² | 5.0 |
| Roasted Soybean Germ | 54 | 10,000 rpm, 7 kg/cm² | 3.4 |

As shown in Table 10, the values of about 3-6 µm were obtained with respect to 50 % grain size for all of the materials used. Refer to the grain size distribution in Fig. 2 as well.

(1) Flaked and defatted soybean germ, (2)flaked and defatted soybean cotyledon and (3) dry and crushed "okara" were ultra-finely crushed using Kriptron KTE- type crusher (Kawasaki Heavy Industry Co.). The crushing conditions and the results are summarized in Table 11.

**[Table 11]**

| No. | Material | Sizes | Rotation Bate | Air Rate (Nm³/min) | Feed Rate (Kg/hr) | Results (50 % Grain Size) |
|---|---|---|---|---|---|---|
| 1 | Flaked and Defatted Soybean Germ | 0.1 - 5 mm long, 0.05 - 1.5 mm thick | 10,400 rpm | 8 | 15 | 6.4 |
| 2 | Flaked and Defatted Soybean Cotyledon | 0.1 - 7 mm long, 0.05 - 1.5 mm thick | 10,400 rpm | 8 | 15 | 6.7 |
| 3 | Dry and Crushed "0kara" | 10 *µ*m (50 % Grain Size) | 10,400 rpm | 8 | 15 | 7.4 |

As shown in Table 11, the values of about 6-8 µm were obtained with respect to 50 % grain size for all of the materials used.

### Example 8

The suspendibility of the ultra-finely crushed powder obtained in Example 7 was evaluaed. After each ultra-finely crushed powder (1 g) was suspended in water (100 ml), the resulting suspension was moved to a measuring cylinder (100 ml) and allowed to stand at a room temperature for 24 hr. The level of the boundary between a transparent layer and an opaque suspension layer was recorded. For comparison, suspendibility of each of the powder crushed with the Pinmill-type crusher was measured in the same way.

As shown in Fig. 12, the boundary level of the crushed powder having 50 % grain size of several hundreds µm ~ several tens µm become 10 ml or less after 24 hr. On the other hand, the ultra-finely crushed powder having 50 % grain size of about 3 µm ~ 6 µm according to the present invention kept its boundary level around 95 ml even after 24 hr, demonstrating that its precipitating rate has been significantly decreased while its suspendibility has been significantly improved.

For comparison, the corn fiber crushed into particles of 100 microns or less with the colloid mill-type mass colloider in the wet state described the Japanese Patent Publication No.Hei.3-69270 was also subjected to the same experiment to show the boundary level of 18 ml.

**[Table 12]**

| Ultra-finely Crushed Powder | Crushed Powder With Jetmill 50% Grain Size (*µ*m) | Level of the Boundary after 24 hrs (ml) | Crushed Powder With Pinmill 50% Grain Size (*µ*m) | Level of the Boundary after 24 hrs (ml) |
|---|---|---|---|---|
| Soybean Hull | 6.0 | 9 3 | 95 | 7 |
| Dry "Okara" | 3.1 | 93 | 93 | 8 |
| Corn Germ Cake | 5.0 | 9 7 | 3 6 1 | 6 |
| Roasted Soybean Germ | 3.4 | 97 | 54 | 5 |

### Example 9

The ultra-finely crushed powder obtained in Example 7 was used as food stuff.
(1)Hamburger: The difference in taste was compared and evaluated between a control hamburger and a test one comprising the ultra-finely crushed powder of soybean hull having 50 % grain size of 6.0 µm.
Mixed ground meat of pork and beef 40.1 (parts); soybean protein 2.8(parts); water 11.5(parts); soybean oil 2.8(parts); onion 17.2(parts); bread crumb 8.6(parts); milk 8.6(parts); egg 7.2(parts); salt 1.0(parts); pepper 0.06(parts); and nutmeg 0.05 (parts)were kneaded, shaped and heated to give a control product. A test product was prepared following the same recipe except that the soybean protein 0.5 was replaced with the same amount of the above ultra-finely crushed powder of soybean hull.
According to a sensory test by a panel consisting of eight persons, no panelist felt roughness for the test product, and the test product was evaluated to be superior to the control one in both hardness and no paste-like feeling. Furthermore, they evaluated that the test product was superior to the control one in binding property and shape-holding capability. (Evaluation: 1(bad) - 5(excellent))

**[Table 13]**

| | Results of Sensory Test | | |
|---|---|---|---|
| | Hardness | No Paste-like Feeling | Total Feeling |
| Control Product | 2.8 | 1.5 | 2.7 |
| Test Product | 3.1 | 1.7 | 3.1 |

(2) Beverage: A test product was prepared by adding the ultra-finely crushed powder of "okara" (50 % grain size: 3.1 µm) to commercial orange juice to a final concentration of 1%. On the other hand, a control product was prepared by adding dry "okara" crushed with the Pinmill type crusher (50 % grain size : 93 µm) to commercial orange juice to a final concentration of 1%. The results obtained in the sensory test by the panel are summarized in Table 14.
(Evaluation: 1(bad) - 5(excellent))

**[Table 14]**

| | Results of Sensory Test | | |
|---|---|---|---|
| | Feeling in Throat | Roughness | Feeling in Drinking |
| Control Product | 1.5 | 1.2 | 1.2 |
| Test Product | 4.2 | 4.5 | 4.5 |

As shown in Table 14, the ultra-finely crushed powder of "okara" (50 % grain size : 3.1 µm) is evaluated to be superior to the dry "okara" crushed with the Pinmill type crusher(50 % grain size:93 µm) in the sensory test.
(3) Ice cram: A test product was prepared in a conventional manner by mixing fresh cream 200 ml, milk 100 ml, roasted soybean germ crushed ultra-finely (50 % grain size: 3.4 µm) 6 g, sugar 30 g, two pieces of egg yolk, and vanilla essence 0.35 ml. The resulting nice-smelling ice cream had no roughness and gave completely the same feeling on our tongue as commercially available ice cream comprising no roasted soybean germ crushed ultra-finely.

### Example 10

A culture medium (40 ml, pH 7.3) comprising 5.0 g of the ultra-finely crushed powder of soybean hull (50 % grain size: 6.0 (µm) obtained in Example 7, glucose 0.1 g, KH₂PO₄ 0.4g, MgSO₄ .7H₂O 0.05 g, soy milk 130 mg, FeSO₄ .7H₂O 1 mg, VB₁ 500 µg and biotin 500 µg was put into a shaking flask (500 ml), capped with a cotton cap, sterilized at 120 ° C for 30 min and subjected to a shaking culture (35 ° C, 113 rpm) for one month.

No growth of bacteria was observed, showing that the sterilization had been sufficiently done. On the other hand, in the case where the ultra-finely crushed powder of soybean hull (50 % grain size: 95 µm) was used instead of the above ultra-finely crushed powder of the present invention in the same way, the growth of bacteria was observed. This result shows that the sterilization was not sufficiently done.

### Example 11

Various kinds of soybean hull (50 g each) having different 50 % grain size were suspended in water (300 ml), and stirred at a room temperature for 30 min to give water soluble extract. Upper liquid layer was taken out with centrifugation, lyophilized and weighed to give a yield of the extract. The results are shown in Table 15.

**[Table 15]**

| Soybean Hull 50 % Grain Size | Yield of Extract |
|---|---|
| 6 µm | 14% |
| 37 µm | 10% |
| 95 µm | 8% |
| 1∼3 mm | 5% |

As shown in Table 15, the soybean hull powder crushed ultra-finely up to 50 % grain size of 6.0 µm according to the present invention has been significantly improved in the yield of the extract compared with the powder having a larger grain size.

### Example 12

The ultra-finely crushed powder of soybean hull (50 % grain size: 3.4 µm) obtained in Example 7 (26 (parts)), corn protein zein 19 (parts), salt 0.5 (parts), ethanol 33 (parts) and water 22 (parts) were mixed well, shaped in a cast and baked at 150 ° C for 5 min to give a tray of 10 cm in diameter, 3 cm high, and 2.5 mm thick. The resulting tray had a smooth surface and enough strength to be used as a tray for food.

For comparison, the soybean hull crushed with the Pinmill type crusher five times (50 % grain size: 12 µm) was used in the same composition and way as above to give a comparative tray. The comparative tray was inferior to the above tray in brightness and strength.

Accordingly, it is shown that the ultra-finely crushed powder (50 % grain size: 3.4 µm) is more advantageous as filler for the production of the food tray than the conventionally used powder (50 % grain size: 12 µm).

### Industrial Applicability

As shown by the above examples, the physicochemical properties that are not sufficiently apparent in the whole tissues have appeared in the fractions according to the present invention.

Accordingly, the present fraction as such may be used, for example, as food stuff or food component for various kinds of food (confectionery, bread, desserts such as ice cream, source, retort pouched food, oil and fat food, emulsified food, liquid food, dietary fiber-enriched food, low-calorie food, low-fat food, nutritional component-enriched food, animal food product and marine product and the like), feed, and material for chemical products. The fraction may be also used as a starting material for effectively extracting specific components such as oil and fat or protein based on the difference in the contents of chemical components among the fractions.

The ultra-finely crushed powder according to the present invention has various excellent properties when used as food stuff or food component, such as smooth feeling on tongue, water-holding capacity and suspendibility in beverage. Furthermore, it shows excellent properties in extracting the contents therein, excellent properties as the component in a culture medium for bacteria in fermentation and brewing, such as excellent sterilization efficiency and transportation efficiency, and excellent properties as the filler for shaping the containers.

## Claims

1. A method for producing a fraction derived from the fine structure portions of a specific tissue of oilseeds or cereals, comprising crushing and classifying material of the specific tissue.

2. A method according to Claim 1 wherein the specific tissue is hull.

3. A method according to Claim 2 wherein the hull is selected from the group consisting of soybean hull, corn outer hull, rape seed hull, sesame seed hull and husk.

4. A method according to Claim 1 wherein the specific tissue is germ.

5. A method according to Claim 4 wherein the germ is selected from the group consisting of soybean germ, corn germ, wheat germ, and rice germ.

6. A method according to Claim 1 wherein the specific tissue is oil and fat extraction cake or protein extraction cake.

7. A method according to Claim 6 wherein the oil and fat-extraction cake or protein-extraction cake is selected from the group consisting of defatted rice bran, corn germ cake, dry "okara" or soybean pulp, rape seed cake and sesame seed cake.

8. A method according to one of Claims 1-7 wherein the classification is carried out based on the difference in specific volume or grain size of the crushed fine structure.

9. A method according to one of Claims 1-7 wherein the material is the fraction obtained by the method according to one of Claims 1-7.

10. A fraction derived from the fine structure portions of a specific tissue of oilseeds or cereals, which is obtained by the method according to one of Claims 1-9.

11. An ultra-finely crushed powder having 50 % grain size of 6 µm or less, which is obtained from the material of a specific tissue of oilseeds or cereals.

12. An ultra-finely crushed powder according to Claim 11 wherein the material is selected from the group consisting of soybean hull, dry "okara" or soybean pulp, soybean germ, corn germ cake, corn outer hull, rapeseed cake, and wheat germ.

13. A method for producing ultra-finely crushed powder having 50 % grain size of 10 µm or less, comprising crushing material of the specific tissue of oilseeds or cereals in a dry state.

14. A method for producing ultra-finely crushed powder according to Claim 14 wherein the crushing is carried out in the dry state by using high-speed compressed air or high-speed compressed gas.

15. Food stuff comprising the ultra-finely crushed powder according to Claim 11 or 12, or the ultra-finely crushed powder obtained by the method according to Claim 13 or 14.

16. Culture medium for bacteria comprising the ultra-finely crushed powder according to Claim 11 or 12, or the ultra-finely crushed powder obtained by the method according to Claim 13 or 14.

17. Filler for shaping containers comprising the ultra-finely crushed powder according to Claim 11 or 12, or the ultra-finely crushed powder obtained by the method according to Claim 13 or 14.
